# EUROPEAN PATENT SPECIFICATION

(11) **EP 1 699 468 B1**
(45) Date of publication and mention of the grant of the patent: **18.04.2007**
(21) Application number: 04798745.8
(22) Date of filing: 18.11.2004
(51) Int. Cl.: A61K 31/728, A61K 33/30, A61P 25/28

(54) **PHARMACEUTICAL COMPOSITION COMPRISING A ZINC-HYALURONATE COMPLEX FOR THE TREATMENT OF MULTIPLE SCLEROSIS**
PHARMAZEUTISCHE ZUSAMMENSETZUNG MIT EINEM ZINK/HYALURONAT-KOMPLEX ZUR BEHANDLUNG VON MULTIPLER SKLEROSE
COMPOSITION PHARMACEUTIQUE COMPRENANT UN COMPLEXE ZINC-HYALURONANE, DESTINEE AU TRAITEMENT DE LA SCLEROSE EN PLAQUES

(30) Priority: 20.11.2003 HU 0303779
(43) Date of publication of application: 13.09.2006
(73) Proprietor: RICHTER GEDEON VEGYÉSZETI GYÁR RT, 1103 Budapest (HU)
(72) Inventor: BALOGH, György, Tibor, H-1172 Budapest (HU); ILLÉS, János, H-1112 Budapest (HU); BOROS, András, H-1024 Budapest (HU); FORRAI, Gáborné, H-1162 Budapest (HU); SZÉKELY, Ákosné, H-1204 Budapest (HU)
(86) International application number: PCT/HU2004/000107
(87) International publication number: WO 2005/049047

(56) References cited:
- EP-A- 1 166 788
- EP-A- 1 369 119
- WO-A-90/10020
- WO-A-97/11710
- WO-A-98/48815
- SZABO I ET AL: "[Effect of hyaluronic acid on cryoagglutinins in experimental allergic encephalomyelitis]" PATOLOGICHESKAIA FIZIOLOGIIA I EKSPERIMENTAL'NAIA TERAPIIA. 1966 SEP-OCT, vol. 10, no. 5, September 1966 (1966-09), pages 41-44, XP008043090 ISSN: 0031-2991 cited in the application
- MISKOLCZY D ET AL: "[THE EFFECT OF HYALURONIC ACID ON THE PROTEIN METABOLISM OF RABBITS WITH EXPERIMENTAL ALLERGIC ENCEPHALOMYELITIS.]" MEDICINA EXPERIMENTALIS. INTERNATIONAL JOURNAL OF EXPERIMENTAL MEDICINE. 1963, vol. 50, 1963, pages 307-312, XP008043089 ISSN: 0258-2589
- SCHIFFER R B ET AL: "Effects of exposure to dietary nickel and zinc upon experimental allergic encephalomyelitis in SWXJ mice" JOURNAL OF TRACE ELEMENTS IN EXPERIMENTAL MEDICINE 1996 UNITED STATES, vol. 9, no. 1, 1996, pages 1-9, XP008043103 ISSN: 0896-548X cited in the application

## Description

The invention relates to pharmaceutical compositions for the treatment of multiple sclerosis which comprises a zinc-hyaluronan complex as active ingredient and a pharmaceutically acceptable carrier and/or additive. The process for the preparation of said pharmaceutical compositions as well as the therapeutic use thereof for the treatment of multiple sclerosis are also within the scope of the invention.

The hyaluronan (HA) is a homopolymer of the glucosaminoglycan type built up of repeated N-acetylglucosamin-glucuronic acid disaccharide units of the formula (I).

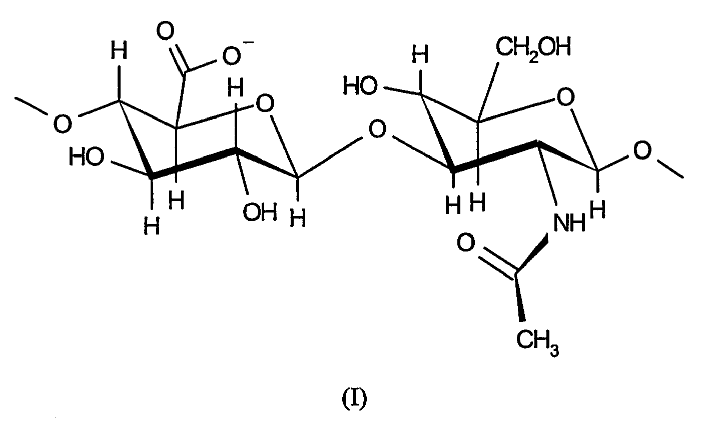

In the HA the monosaccharides are β(1→3) linked, whereas the disaccharide units are β(1→4) linked, thus forming a linear polysaccharide with alternating β(1→3) and β(1→4) linkages.

The HA in living organisms occurs as a salt formed with a cation, usually sodium, and its molecular weight may range from 10-20 kDa to several thousands kDa. The presence of carboxyl group in the glucuronic acid moiety of the HA and the carbonyl and amino groups in the N-acetyl group of the glucosamine, as well as the hydroxyl groups being present facilitate the formation of several hydrogen bridges. Due to these intramolecular hydrogen bonds and to the hydrogen bridges formed via interactions between HA and the water being present in the biological systems, HA has a complicated three dimensional structure (C. L. Hew et al., Eur. J. Biochem. 203, 33-42. (1992); Q. Liu et al. J. Am. Chem. Soc 118, 12276-12286. (1996)). As a consequence of its exceptional water binding ability even relatively dilute aqueous solutions of HA show high viscosity. In aqueous solutions its rheologic properties are highly dependent upon the size of the molecule; e. g. in 1% aqueous solution HA of the 1000 kDa molecular size has 3000 mPa viscosity, whereas HA of the 4000 kDa molecular size has 400000 mPa viscosity in similar concentration (H. B. Wik and O. Wik: Rheology of Hialuronan, The Chemistry, Biology and Medical Applications of Hialuronan and its Derivatives (ed. T. C. Laurent) pp. 25-32. Portland Press, London, (1998)). Accordingly, the two most important physical features of HA are the viscosity and the molecular size thereof.

As a main component of the extracellular matrix HA is present in all parts of the body. Certain organs and tissues (connective tissues, skin, synovial fluid, vitreous humour, and blood vessel wall) contain HA in an increased amount. It has long been thought that the biological role of HA derived from its physical properties. For instance it can provide mechanical protection to joints by virtue of its rheological nature. Owing to its exceptional water binding ability HA can control the water balance through its osmotic pressure and by offering resistance to flow. HA also plays an important role in filling up the interstitium and protects cells from different physical impacts. Recent investigations showed that the interaction between HA and certain macromolecules present in the body could be brought into connection with several physiological processes. Examples of such macromolecules are the proteoglycanes (aggrecan, versican, brevican, etc.) which are situated in the extracellular matrix and have the main task to occupy the space between cells and to facilitate material transport. Macromolecules entering into interactions with HA can be intracellular transmembrane proteins (CD44, RHAMM), as well as receptor proteins present in the cytoplasm (C1q, P-32, TSG-6, etc.). Through the proteins mentioned above HA plays an important role in several control processes taking place at cell or body level.

The molecular size and the concentration of HA in aqueous solution have - as it is the case with the physical properties- a considerable influence on its biological effect. Thus, depending on the size of the molecule, HA may exert either a positive or a negative effect on the same cellular process. Similar change of effect was seen when the HA concentration of the solution had been altered. The desired optimal effect can obviously be achieved when both parameters are simultaneously taken into consideration (E. A. Balázs, The Chemistry, Biology and Medical Applications of Hialuronan and its Derivatives (ed. T. C. Laurent) pp. 185-204. Portland Press, London (1998)).

Since HA participates in the physiological processes mentioned above, it can successfully be used in several fields of therapy (wound-healing, treatment of chronic inflammation, ophthalmic surgery).

The range within which HA is applicable in the human therapy - beyond those mentioned above - can be widened by modifying the structure chemically. In this respect two main trends are known. According to one of them cross-linkages are established between two distant positions of the HA molecule by using an aliphatic compound (usually a dihydrazide) to form a hydrogel. The cross-linkages cause an increase in the viscoelasticity of the chemically modified HA resulting in greater resistance to degradation effects occurring in the body. It is to the advantage of patients with rheumatoid arthritis treated to regain the synovial fluid or of those having postoperative adhesion. In another important strategy active agents which are difficult to absorb or are to be passed specifically to the location of effect are chemically bound to the HA (e. g. taxol, pilocarpine, insulin). In these cases HA causes improved absorption of the active agents bound to HA and assists specific arrival of the matter to the target place, respectively.

Complexes of HA formed with zinc and cobalt are described in EP 413016 (Burger et al., 1989), and of the two the Zinc-HA is used as active agent in compositions for wound treatment. Further studies of the active agent showed that due to the presence of zinc possibilities for therapeutic use can be broadened, since the zinc complex has new or more expressed effects compared with the sodium-HA salt (J. Illés et al., Acta Pharm. Hung. 72, 15-24 (2002)). Among others such effects are the increased antioxidant activity of the zinc-HA (Gy. T. Balogh et al., Arch. Biochem. Biophys. 410, 76-82. (2003)); the inhibitory effect on tissue damaging enzymes (matrix metalloproteinases, particularly the MMP-9) which are produced in increased amount by invasive cells, while the sodium-HA salt does not show the latter effect (WO 00/53194, Illés et al.1999). The gastroprotective effect (treatment of peptic ulcer) of the zinc-HA is disclosed in published WO 98/48815 international patent application, antimicrobial effect of the zinc-HA active agent is described in published WO 98/10773 international patent application.

Multiple sclerosis (MS) is a chronic autoimmune disease of the central nervous system. The disease first manifests itself between the ages of 15 and 40, and the rate of occurrence in women is double (Duquette et al., Can. J. Neurol. Sci. 19, 466-71. (1992)). The pathology of the disease is complex and in several respects unclear. While there is a clear evidence of genetic susceptibility and disturbance of the immunological processes, the role of an environmental factor (e. g. certain viral infections) is uncertain (Stipindonk et al., J. Neuroimmunol. 105, 46-57. (2000)). Clinical forms of the disease are greatly varied, most of the cases are of the relapsing-remitting type, all the same. MS, a neurodegenerative disease manifested by the disorders of the sense and locomotor organs as well as the autonomous system, develops slowly, undermines mental and affective abilities and ultimately leads to severe disability. Histopathology shows first foci of inflammation in the central nervous system, later demyelination and decline of axon appear (Ewing et al., Immunol. Cell Biol. 76, 47-54. (1998)). Lasting clinical symptoms are induced by degradation of the myelin sheath and deterioration of the oligodendrocyte resulting in a plurality of nerve injuries (Fu et al., Brain 121, 103-113. (1998)). According to the present state of knowledge in medicine, MS is an incurable disease. In everyday practice non-specific immunsuppresive substances (corticosteroids, cytostatic agents) or more specific compositions which modify the course of the disease (beta-interferons, glatiramer acetate) are used. Applicability of said pharmaceuticals, however, is restricted by low effectiveness and severe side effects.

Up to now eleven drugs have been put on the market which can modify the course of the disease and clinical trials are underway with 24 compositions. The two most advanced compositions, i. e. the Copaxone (copolymer-1 or glatiramer acetate) and the Rebif or Avonex (interferon beta-1a), as well as the interferon beta-1b are beneficial only to one form (the so called benign, or relapsing-remitting form) of the disease - further forms of the sickness are the primary and secondary progressive forms as well as the malignant MS - (Otten et al., Comparsion of drug treatments for multiple sclerosis. Ottawa: Canadian Coordinating Office for Health Technology Assessment. (1998); Parkin et al., Health Technol. Assess. 2(4) (1998)), and they can reduce the frequency of relapses only by about 20-30 % (Khan et al., CNS Drugs 16(8), 563-578. (2002)). Moreover both of the compositions possess several undesirable side effects. In the case of the compositions containing interferon beta-1 influenzalike symptoms, skin reactions and local pain - as consequences of the subcutaneous administration - may occur, and disorders of the blood picture, increased level of the liver enzymes, as well as personality disorders may also appear (Eber et al., Lancet 352, 1498-1504, (1998); Jacobs et al., Mult. Scler. 1, 118-135. (1995)). In the case of copolymer-1 the most characteristic side effects, again are the skin reactions and local pain - as consequences of the subcutaneous administration, further, - redness of face, pressure in the thorax, palpitation and laboured respiration may occur (Johnson et al., Neurology 45, 1268-1276, (1995); Bornstein et al., N. Engl. J. Med. 317, 408-414. (1987)). Although these drugs more or less reduce the inflammation appearing in the CNS (in the primary form of the disease), they do not affect the progression of the disease (Clegg, Health Technol. Assess. 4(9) (2000)).

The only adequate and widely used model available for purposes of preclinical investigations of multiple sclerosis is the experimental autoimmune encephalomyelitis (EAE) (Mokhatarian et al., Nature 309, 356-358. (1984); Raine et al., Lab. Invest. 31, 369-380. (1974)), an inflammatory disease of the central nervous system transmitted by CD4⁺ T-lymphocytes. Although the clinical complexity of the model is moderate compared with the disease investigated, its use is unavoidable in a research aimed at MS. Preclinical tests of Copolymer-1 (Copaxone) and interferon beta-1 already approved for human treatment, as well as those of the new drugs under development were/are carried out using this model (Popovic et al., Ann. Neurol. 51, 215-223. (2001); Stanislaus et al., Neurosci. Lett. 333, 167-170. (2002)). Of the several EAE models it is the chronic relapsing-remitting type (CR EAE) which proved the most suitable both for testing the demyelination, a feature of MS, and for studying the immunological processes. As for the clinical course of the disease, CR EAE is the most comparable to MS (Wekerle et al., Ann. Neurol. 36, 47-50. (1994)). That's why in our experiments CR EAE provoked by myelin oligodendrocyte glycoprotein (MOG) in mice has been studied (Amor et al., J. Immunol. 153, 4349-4356. (1994)). In the middle of the 1960s the effect of hyaluronan sodium salt was investigated by Romanian research workers on EAE model. They found that the Na-HA salt given intramuscularly did not inhibit, but in certain cases did assist the development of EAE in rabbits (Szabó et al., (1966) Patol. Fiziol. Eksp. Ter. 10(5), 41-44.; Miskolczy et al., (1965) Stud. Cercet. Neurol. 10(5), 493-497.). Neither showed success the investigations in EAE model performed with different zinc salts given intraperitoneally or orally (Schiffer et al., (1996) J. Trace Elem. Exp. Med. 9(1), 1-9.; Penkowa and Hidalgo (2000) Glia 32(3), 247-263.).

In the published WO 97/11710 international patent application tests for the influence on immunological processes are disclosed; said tests utilize Na-hyaluronate, hyaluronic acid and a conjugate thereof with different molecular weights. Particularly, proliferation of mouse T-cells as well as inhibitory effect in a graft vs. host reaction were proved, wherein a graft of cardiac muscle tissue taken from a donor of another species of mice was used. Although the possible use of hyaluronic acid and Na-hyaluronate, respectively, in multiple sclerosis is mentioned in the specification, there is no experimental evidence supporting this hypothesis. We also tested an associate formed between hyaluronic acid and sodium (Na-hyaluronan) to check up the above statement but it didn't exert any influence on the EAE model, specific for MS, as shown in Table 4 of this application.

Taking into consideration on one hand that the prior art drugs had harmful side effects and that the Na-HA salt was ineffective, and on the other hand that the Zn-HA complex gave promising results in different fields of therapy, our aim was to test the Zn-HA complex for MS using the most comparable EAE animal model.

We have surprisingly found that while both the Na-HA and the Zn²⁺ were ineffective alone, various doses of Zn-HA with different molecular weights inhibited the EAE induced in the model animals. This effect in optimised conditions was significant.

Accordingly, the invention relates to a pharmaceutical composition for the treatment of multiple sclerosis, which comprises a zinc-hyaluronan complex, preferably a zinc-hyaluronan complex with a molecular weight of 800-1200 kDa, as active ingredient and a pharmaceutically acceptable carrier and/or additive.

Another object of the invention is a use for the preparation of a pharmaceutical composition of a mixture containing a zinc-hyaluronan complex active agent in admixture with a carrier and/or additive conveniently used in medicine to obtain the composition. Said pharmaceutical composition preferably is a solution, suitably a solution for injection or infusion.

A further object of the invention is the use of a zinc-hyaluronan complex for the preparation of a medicament for the treatment of multiple sclerosis.

Said treatment can be carried out e. g. via intravenous administration using a zinc-hyaluronan complex having a molecular weight of 800-1200 kDa.

In our experiments Zn-HA complexes with different molecular weights in different doses were sub cutane (s. c.) administered to the EAE model and the animals were tested for inhibitory effect from day 5 to day 22 after EAE had been induced. Namely, Zn-HA complexes of four different average molecular weights (10, 50, 200 and 800-1200 kDa (the latter one hereafter marked as HMW)) in three different doses (1, 10 and 50 mg/kg) were investigated to determine that Zn-HA of which molecular weight and in which dose shows optimal effect in the selected type of the EAE model. The results are shown in Table 1 below.

**Table 1**

| Zn-HA complexes with different MW | Test results in the case of 3 different doses | | | | | |
|---|---|---|---|---|---|---|
| | 1 mg/kg | | 10 mg/kg | | 50 mg/kg | |
| | Inhibition % | No. of tests | Inhibition % | No. of tests | Inhibition % | No. of tests |
| 10 kDa Zn-HA | 4.2+12.5 | 3 | 32.8±11.7 | 6 | 43.8±23.9 | 3 |
| 50 kDa Zn-HA | -12.7 | 2 | 34.8±16.6 | 6 | 33.2 | 1 |
| 200 kDa Zn-HA | -2.2±53.2 | 3 | 25.2±11.3 | 6 | 24.2±21.9 | 3 |
| HMW Zn-HA | 24.5 | 2 | 41.3±7.1 | 9 | 32.9 | 1 |

Taking into consideration both the extent of inhibition and the deviation from the mean, HMW Zn-HA complex at 10 mg/kg dose was the most effective (41.3±7.1 % inhibition).

Next, the possible effect of the HMW Zn-HA (10 mg/kg in 200 µl of physiological saline, subcutane) on the time of appearance of clinical symptoms has been investigated.

Tests were carried out on groups of mice (n=6-10 mice/group) using two types of treatment: (i) prophylactic, wherein administration happens from day 5 to day 22 after inoculation of MOG, or in a single case from day 9-10 to day 28 after that (marked with # in tables 2 and 3); and (ii) therapeutic treatment, wherein administration of the Zn-HA happened from day 13 to day 28 after the EAE had been induced (marked with * in tables 2 and 3).

To ascertain effectiveness 9 independent tests were performed with the results shown in tables 2 and 3. The mice were given 10 mg/kg HMW Zn-HA s. c. daily and clinical data thereof were obtained from day 10 to day 28 and day 5 to day 22, respectively, after the EAE had been induced. The results were calculated from the average daily clinical data to give the average summed up clinical data (ASC).

**Table 2**

| Ser. No. of experiment | n | | Appearance time of the disease counting from the inoculation day | | Incidence (%) | | ASC (10-28) | |
|---|---|---|---|---|---|---|---|---|
| | control | treated | control | treated | control | treated | control | treated |
| 1* | 10 | 7 | 13 | 20 | 100.0 | 85.7 | 24.2 | 12.6 |
| 2 | 8 | 10 | 11 | 10 | 100.0 | 40.0 | 23.7 | 10.4 |
| 3^{#} | 10 | 8 | 11 | 13 | 70.0 | 75.0 | 21.8 | 14.9 |
| 4 | 7 | 7 | 13 | 13 | 85.7 | 71.4 | 26.6 | 18.3 |
| | | | | | | | ASC (5-22) | |
| 5 | 7 | 8 | 11 | 15 | 85.7 | 37.5 | 15.2 | 2.6 |
| 6 | 9 | 8 | 15 | 16 | 100.0 | 75.0 | 19.4 | 10.7 |
| 7 | 6 | 6 | 17 | 17 | 83.3 | 50.0 | 12.0 | 6.8 |
| 8 | 9 | 10 | 15 | 16 | 90.0 | 90.0 | 20.2 | 18.9 |
| 9 | 9 | 15 | 13 | 16 | 77.7 | 93.6 | 18.4 | 13.3 |

**Table 3**

| | Administered substance | | Inhibition % |
|---|---|---|---|
| | Carrier (n=75) | HMW Zn-HA (n=79) | |
| | (Mean ± deviation) | (Mean ± deviation) | |
| ASC | 20.2±1.5 | 12.1±1.7 | 41.3* |
| Appearance time of the disease counting from the inoculation day | 13.1±0.5 | 14.9±0.9 | - |
| Incidence (%) | 88.0±3.5 | 68.6±7.0 | 22.1^{#} |

| | | | |
|---|---|---|---|
| n = number of animals participating in the experiment Carrier: physiological saline | | | |

Tables 2 and 3 show that by the administration of HMW Zn-HA at 10 mg/kg s. c. dosage a significant inhibition in the severity of the clinical symptoms of induced EAE has been achieved (41.3±7.1 % inhibition and 12.1 ASC value were obtained at p<0.004 by the Mann-Whitney's U test) and the incidence of the clinical symptoms has also been reduced by 22 % (68.6 % for the complex and 88% for the control, at p<0.012 according to the t-test). On the other hand, the timing of the treatment (whether it is prophylactic or therapeutic) causes no considerable change in the effect of Zn-HA exerted on the EAE.

In previous experiments we have found that - contrary to the HMW Zn-HA - the zinc chloride containing ZN²⁺ in an amount equivalent to 10 mg/kg HMW Zn-HA (i. e. 0.8 mg/kg Zn²⁺) did not show significant effect on the EAE model; neither did the Na-HA salt, as it was mentioned in the introductory part of the present application (for the results see Table 4 below).

**Table 4**

| | Carrier | HMW Na-HA | Carrier | ZnCl₂ | Carrier | HMW Zn-HA |
|---|---|---|---|---|---|---|
| | (n=7) | (n=14) | (n=7) | (n=14) | (n=75) | (n=79) |
| ASC | | | | | | |
| (treatment from day 5 to day 22) | 15.7±3.7 | 14.2±1.9 | 13.1±3.3 | 14.2±3.3 | 20.2±1.5 | 12.1±1.7 |

| | | | | | | |
|---|---|---|---|---|---|---|
| n = number of animals participating in the experiment carrier: physiological saline | | | | | | |

A pharmaceutical composition containing the Zn-HA complex active agent according to the present invention can be administered in any traditional route, e. g. orally, parenterally, through the oral mucous membrane, sublingually, through the nasal mucous membrane, rectally, transdermally, intravenously (infusion) or intramuscularly. The pharmaceutical composition for purposes of oral administration may be in liquid or in solid form, e. g. in the form of a syrup, suspension, emulsion, tablet, capsule and lozenge. Liquid compositions, such as suspensions or solvents contain the Zn-HA complex active ingredient in a suitable liquid carrier, e. g. in an aqueous solvent, such as water, ethanol or glycerol, or in a non-aqueous solvent, such as polyethylene glycol or an oil, or a mixture thereof. The composition may also contain a suspending agent, a preservative, flavouring or colouring agents. When the pharmaceutical composition is prepared in the form of tablet, any suitable carrier conveniently used for the preparation of solid compositions may be utilized. Examples of such carriers are magnesium stearate, starch, lactose, sucrose, cellulose.

Solid compositions of the capsule form may be prepared in manner known per se. For instance a pill containing the active ingredient together with known carriers may be filled into a hard gelatine capsule; or a dispersion or suspension of the active agent together with any carrier conveniently used in medicine (e. g. gum Arabic, cellulose, silicates or oils) can be encapsulated in soft gelatine.

The typical parenteral compositions are solutions or suspensions which contain the Zn-HA complex and a sterile aqueous carrier or an oil acceptable for parenteral use, such as polyethylene glycol, polyvinyl pyrrolidone, lecithin, arachis oil or sesame oil. According to another pattern the solution obtained is lyophylized and dissolved again in a suitable solvent directly before use.

Compositions for administration through the nasal mucous membrane are formulated in manner known per se to give an aerosol, drops, gels or dusts. The aerosol composition contains the active ingredient in dissolved or finely dispersed form in a pharmaceutically acceptable aqueous or non-aqueous solvent which then is filled into a container under sterile conditions. Said container can be a cassette or container equipped with a nozzle and may contain single or multiple dose(s). Compositions for administration through the oral mucous membrane or for sublingual absorption can be in tablet, pill or pastille form. Said compositions contain the active ingredient in admixture with a carrier, such as sugar, gum Arabic, tragacanth gum, gelatine, glycerol or the like.

Compositions for rectal use are prepared in manner known per se in the form of suppositories which besides the active ingredient contain also a carrier, usually cocoa butter. Compositions for topical use are e. g. ointments, gels and plasters.

For purposes of investigations the Zn-HA active agent was dissolved in physiological saline and was used in the concentration range of 0.1 to 5 mg/ml.

Further, any aqueous solution containing the Zn-HA active ingredient in physiologically compatible concentration at a pH maintained by a buffer at a physiologically compatible value can be used. Other non-aqueous solvents which are physiologically acceptable and in which the active ingredient is properly soluble may also be used. Examples of such solvents are the ethanol, propylene glycol, animal and vegetable oils and the like, as well as the aqueous mixtures thereof Such solutions may contain the Zn-HA in a concentration range of 0.01-100 wt %.

Additives having no direct influence on the potency of the active ingredient are also applicable; examples of such additives are buffer solutions, preservatives and additives which assist absorption. Examples of water-soluble preservatives are sodium bisulfite, sodium bisulfate, sodium thiosulfate, benzalkonium chloride, chlorobutanol, timerosal, methylparaben, polyvinyl alcohol, phenylethyl alcohol and the like. The concentration of said additives in the aqueous solution can be of 0.001-5 wt %. Examples of water soluble buffer agents are the sodium carbonate, sodium borate, sodium phosphate, sodium acetate, sodium bicarbonate and the like. The maximum concentration of these additives in the aqueous solution can be 5 wt % as compared to that of the active ingredient.

### Example 1

In the studies summarized in Tables 1 to 4 each mouse used as EAE model received 200 µl s. c. injection each; the injection contained the active ingredient or the carrier (0.9 % sodium chloride aqueous solution). To obtain the doses given in terms of mg/kg bodyweight above solutions of different concentration were prepared as follows:

### For the 1 mg/kg dose:

A solution composition containing 0.1 g active ingredient (Zn-HA complex, Na-HA or Zn-Cl₂) per 1000 ml solution; e. g.:
0.1 g Zn-HA complex and
0.9 % sodium chloride solution in distilled water to 1000 ml.

### For the 10 mg/kg dose:

A solution composition containing 1.0 g active ingredient (Zn-HA complex, Na-HA or Zn-Cl₂) per 1000 ml solution; e. g.:
1.0 g Zn-HA complex and
0.9 % sodium chloride solution in distilled water to 1000 ml.

### For the 50 mg/kg dose:

A solution composition containing 5.0 g active ingredient (Zn-HA complex, Na-HA or Zn-Cl₂) per 1000 ml solution; e. g.:
5.0 g Zn-HA complex and
0.9 % sodium chloride solution in distilled water to 1000 ml.

## Claims

1. Use of zinc-hyaluronan complex for the manufacture of a medicament for the treatment of multiple sclerosis.

2. Use of zinc-hyaluronan complex according to claim 1, wherein the zinc-hyaluronan complex has a molecular weight of 800-1200 kDa.

3. Use of zinc-hyaluronan complex according to claim 1 or 2, wherein the medicament manufactured is in the form of a solution

4. Use of zinc-hyaluronan complex according to any of claims 1 to 3, wherein the medicament manufactured is in the form of injection, for subcutaneous, intramuscular or intravenous administration.

5. Use of zinc-hyaluronan complex according to any of claims 1 to 3, wherein the medicament manufactured is in the form of infusion.

## Patentansprüche

1. Verwendung des Zinkhyaluronan-Assoziats (-Komplexes) zur Herstellung eines Medikamentes für die Behandlung von Multiple Sklerose.

2. Verwendung des Zinkhyaluronan-Assoziats (-Komplexes) gemäss Anspruch 1, worin das Zinkhyaluronan-Associat (-Komplex) ein Molekulargewicht von 800-1200 KDa aufweist.

3. Verwendung des Zinkhyaluronan-Assoziats (-Komplexes) gemäss Anspruch 1 oder 2, worin das Medikament in Form einer Lösung ist.

4. Verwendung des Zinkhyaluronan-Assoziats (-Komplexes) gemäss Anspruch 1 bis 3, worin das Medikament in Form einer Injektion für subkutane, intramuskuläre oder intravenöse Anwendung ist.

5. Verwendung des Zinkhyaluronan-Assoziats (Komplexes) gemäss Anspruch 1 bis 3 worin das Medikament in Form einer Infusion ist.

## Revendications

1. Utilisation d'un complexe d'hyaluronane de zinc pour la préparation d'un medicament pour le traitement de la sclérose en plaques.

2. Utilisation d'un complexe d'hyaluronane de zinc selon la revendication 1, dans laquelle le complexe d' hyaluronane de zinc présente un poids moléculaire de 800-1200 kDa.

3. Utilisation d'un complexe d'hyaluronane de zinc selon la revendication 1 ou 2, dans laquelle le médicament fabriqué est sous la forme d'une solution.

4. Utilisation du'un complexe d'hyaluronane de zinc selon les revendications 1 à 3, dans laquelle le médicament fabriqué est sous la forme d'une injection pour l'administration hypodermique, intramusculaire ou intraveineuse.

5. Utilisation du'un complexe d'hyaluronane de zinc selon les revendications 1 à 3, où le médicament fabriqué est sous la forme d'une perfusion.
